# EUROPEAN PATENT APPLICATION

(11) **EP 3 818 946 A1**
(43) Date of publication of application: **12.05.2021**
(21) Application number: 19829757.4
(22) Date of filing: 02.07.2019
(51) Int. Cl.: A61B 17/00

(54) **DEVICE FOR INJECTING MEDICAL POWDER INSIDE A BODY**

(30) Priority: 02.07.2018 JP 2018125860
(71) Applicant: Nipro Corporation, Osaka-shi, Osaka 531-8510 (JP)
(72) Inventor: SUGAHARA, Yoshikatsu, Osaka-shi, Osaka 531-8510 (JP); NAKAMURA, Yusuke, Osaka-shi, Osaka 531-8510 (JP)
(74) Representative: Bertsch, Florian Oliver
(86) International application number: PCT/JP2019/026257
(87) International publication number: WO 2020/009102

(57) **Abstract**

An internal spray device (10) for medical powder whereby the medical powder is supplied via a powder channel (66) to an spray channel (32) in which pressurized gas flows, and the medical powder and the pressurized gas are sprayed into a body via the spray channel, the device (10) including a conductive member (74) that is exposed to the powder channel, and a static eliminator (76) that is exposed to an external space (80) that is outside the powder channel, wherein the conductive member (74) and the static eliminator are electrically connected.

## Description

### TECHNICAL FIELD

The present invention relates to an internal spray device for medical powder used in a surgical operation or the like. More particularly, the present invention pertains to an internal spray device for medical powder suitably used for spraying and treating a powder for adhesion prevention or hemostatic treatment to a tissue.

### BACKGROUND ART

If a tissue such as an organ damaged by a surgical procedure such as surgery or tissue collection is left unattended, there is a risk that the damaged portion may adhere to the surrounding tissue. Such tissue adhesion causes infertility due to tubal adhesions, intestinal obstruction due to intestinal adhesions, and the like which impose a heavy burden on the patient. This may necessitate peeling of the adhered portion during reoperation and could cause new damage to organs. Therefore, in order to prevent adhesion in a damaged tissue, a film-shaped adhesion preventive material that physically covers a wound portion during surgery or the like has been conventionally used.

Meanwhile, in recent years, the number of cases in which surgical procedures such as surgery are performed under an endoscope is increasing because the burden on the patient can be reduced.

However, it has been difficult to use the conventionally used film-shaped adhesion preventive material during endoscopic surgery or the like. That is, it is conceivable that a film-shaped adhesion preventive material is rolled into a small size and inserted into the body through a trocar, and then the adhesion preventive material is spread inside the body and attached so as to cover the damaged portion. However, there is a problem that the operation under the endoscope becomes complicated, and the film-shaped adhesion preventive material is likely to be cracked, as well as it is difficult to attach the adhesion preventive material to the correct position to cover the damaged portion.

In view of such a problem, the present inventors proposed, for example, in Japanese Unexamined Patent Publication No. JP-A-2017-51545 (Patent Document 1) or the like, an internal spray device for medical powder which is capable of spraying a powdery adhesion preventive material or the like into the body. Thus, by spraying the adhesion preventive material or the like as powder on the treatment site, it becomes possible to more easily perform the treatment with the adhesion preventive material even under the endoscope. Therefore, the present inventors have conducted further research, developed a spray device capable of spraying medical powder more stably, and achieved the present invention.

### BACKGROUND ART DOCUMENT

### PATENT DOCUMENT

Patent Document 1: JP-A-2017-51545

### SUMMARY OF THE INVENTION

### PROBLEM THE INVENTION ATTEMPTS TO SOLVE

The present invention has been developed in view of the above-described matters as the background, and it is an object of the present invention to provide an internal spray device for medical powder with a novel structure which is able to spray medical powder more stably.

### MEANS FOR SOLVING THE PROBLEM

A first preferred embodiment of the present invention provides an internal spray device for medical powder including a spray channel where pressurized gas flows and a powder channel through which the medical powder is supplied to the spray channel so that the medical powder is sprayed together with the pressurized gas via the spray channel into a body, the internal spray device being characterized in that: the internal spray device further includes a conductive member exposed to the powder channel and a static eliminator exposed to an external space outside the powder channel, and the conductive member and the static eliminator are electrically connected to each other.

According to the internal spray device structured following the present preferred embodiment, static electricity generated by rubbing of the medical powder against the powder container in which the medical powder is contained or the powder channel, and/or by rubbing among the powder itself, will be discharged and eliminated, for example, to the external space through the conductive member exposed to the powder channel and the static eliminator. As a result, it is possible to prevent the medical powder from sticking to the powder container or the powder channel, or from agglomerating due to the action of static electricity. This allows the medical powder to be sprayed stably without clogging the powder channel, for example.

A second preferred embodiment of the present invention provides the internal spray device according to the first preferred embodiment, wherein the internal spray device further includes a vibrator mechanism vibrating the powder channel.

According to the internal spray device structured following the present preferred embodiment, the vibration applied to the powder channel by the vibrator mechanism is also applied to the powder container or the like containing the medical powder, whereby the medical powder is delivered from the powder container through the powder channel to the spray channel. Here, due to the vibration applied by the vibrator mechanism, rubbing between the medical powder and the container or the passage, and/or rubbing among the powder itself are more likely to occur, so that static electricity is more likely to be generated. Thus, the effects of the present invention can be excellently obtained.

A third preferred embodiment of the present invention provides the internal spray device according to the first or second preferred embodiment, wherein a member constituting the powder channel is formed of a thermoplastic resin.

According to the internal spray device structured following the present preferred embodiment, the effects of the present invention can be excellently obtained. That is, since the synthetic resin typically has electric insulation properties, the surface thereof is readily charged. Therefore, when the member constituting the powder channel comprises a synthetic resin, static electricity is more likely to be generated due to rubbing against the medical powder. However, by eliminating the static electricity according to the present invention, the medical powder can be stably sprayed. A thermoplastic resin is preferably adopted as the synthetic resin constituting the powder channel, because of its advantage in molding and handling.

A fourth preferred embodiment of the present invention provides the internal spray device according to any of the first through third preferred embodiments, wherein the medical powder comprises an adhesion preventive material.

According to the internal spray device structured following the present preferred embodiment, since the medical powder comprises an adhesion preventive material, it is preferable for the medical powder to be contained in a dry state in a powder container, for example, from the viewpoint of stability or the like. In such a dry state, static electricity is even more likely to be generated due to rubbing between the powder and the container or the passage, and/or rubbing among the powder itself. Thus, the effects of the present invention can be excellently obtained.

### EFFECT OF THE INVENTION

According to the internal spray device structured following the present invention, static electricity generated by rubbing between the medical powder and the powder container or the powder channel, and/or rubbing among the powder itself is eliminated. This prevents the medical powder from sticking to the powder container or the powder channel or from agglomerating, and the medical powder can be sprayed stably without clogging the powder channel.

### BRIEF DESCRIPTION OF THE DRAWINGS

FIG. 1 is a perspective view showing an entirety of an internal spray device for medical powder according to a first practical embodiment of the present invention, in a state in which a pressing part is operated to spray powder.
FIG. 2 is a front view of the internal spray device for medical powder shown in FIG. 1.
FIG. 3 is a top plan view of the internal spray device for medical powder shown in FIG. 1.
FIG. 4 is a cross sectional view taken along line 4-4 of FIG. 3.
FIG. 5 is a perspective view showing a retaining member that constitutes the internal spray device for medical powder shown in FIG. 1.
FIG. 6 is a front view of the retaining member shown in FIG. 5.
FIG. 7 is a right side view of the retaining member shown in FIG. 5.
FIG. 8 is a cross sectional view taken along line 8-8 of FIG. 6.
FIG. 9 is a perspective view showing a conductive member that constitutes the internal spray device for medical powder shown in FIG. 1.
FIG. 10 is a vertical cross sectional view of a retaining member that constitutes an internal spray device for medical powder according to a second practical embodiment of the present invention, showing a state in which a powder container containing the medical powder is attached.

### EMBODIMENTS FOR CARRYING OUT THE INVENTION

Hereinafter, practical embodiments of the present invention will be described in reference to the drawings.

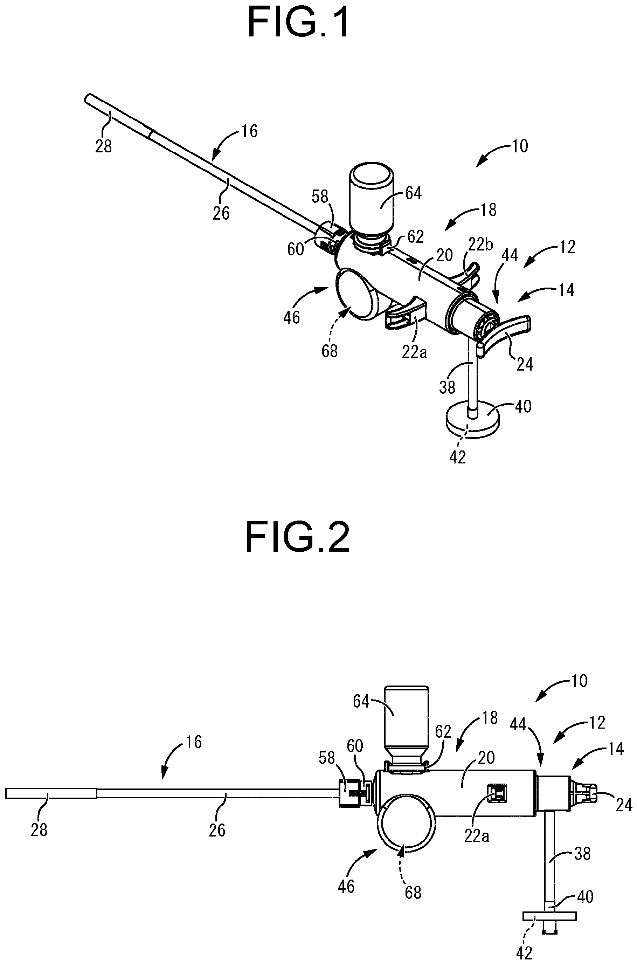

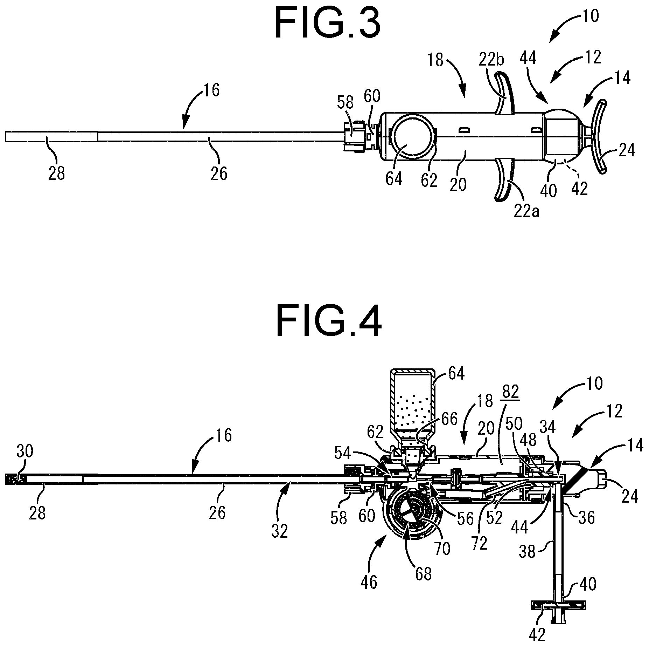

First, FIGS. 1 to 4 show an internal spray device for medical powder (hereinafter, also referred to as a spray device) 10 according to a first practical embodiment of the present invention. The spray device 10 has a roughly syringe shape overall, and by grasping a grasping part 12 and operating a piston-like pressing part 14, the medical powder is sprayed from the distal end of a spray nozzle part 16 together with pressurized gas into a body. In the following description, the axial direction refers to the left-right direction in FIG. 2 in which the spray device 10 extends, and the distal end direction refers to the left direction in FIG. 2, which is the distal end side of the spray nozzle part 16, and the proximal end direction refers to the right direction in FIG. 2, which is the grasping part 12 side. Further, the vertical direction refers to the vertical direction in FIG. 2, and the left-right direction refers to the vertical direction in FIG. 3.

Described more specifically, the spray device 10 is configured such that a device main unit 18 includes a hollow body part 20 and a pressing part 14 attached to the body part 20. The device main unit 18 has a roughly syringe shape overall, and finger hook parts 22a, 22b protruding in mutually opposite directions in the left-right direction are formed at the proximal end portion of the body part 20. In the present practical embodiment, each of the finger hook parts 22a, 22b has a roughly arcuate shape so that the user can easily hook his/her finger during use. The body part 20 has a divided structure in the width direction (vertical direction in FIG. 3) and is formed by overlapping end faces of half cylindrical members and fixing them by bonding or fitting.

Further, the distal end portion of the piston-shaped pressing part 14 is inserted into the proximal end opening part of the body part 20. The pressing part 14 is slidable in the axial direction in the proximal end portion of the device main unit 18. Besides, the pressing part 14 is configured such that the outer diameter dimension of its distal end is larger than the inner diameter dimension of the body part 20 at the proximal end, so that the pressing part 14 does not slip out of the body part 20 to the proximal end side. Besides, the proximal end portion of the pressing part 14 comprises an operating part 24 extending in the left-right direction. In the spray device 10, the grasping part 12 is constituted by including the pressing part 14, the operating part 24, and the finger hook parts 22a, 22b.

On the other hand, at the distal end of the body part 20, there is provided the spray nozzle part 16 extending to the distal end side. The spray nozzle part 16 is constituted by including the structure in which a tubular hard tube 28 is fixed to the distal end side of a soft tube 26 extending in the axial direction. With such a structure, it is possible to easily change the spray direction of the spray nozzle part 16 during surgery by pinching the hard tube 28 with forceps or the like. In the present practical embodiment, a rectifier plate 30 is attached in a housed state to the distal end opening of the hard tube 28. While the shape of the rectifier plate 30 is not limited in particular, in the present practical embodiment, the rectifier plate 30 has a twisted plate shape. The spray fluid is guided along the twisted surface of the rectifier plate 30 and is sprayed to the outside while being rectified in a spiral shape, whereby centrifugal force based on a spiral rotation action is exerted on the medical powder. Thus, the medical powder will be spread and sprayed over a wider range in a roughly uniform manner.

Inside the spray device 10, there is provided a spray channel 32 extending from the end of the pressing part 14 to the distal end of the spray nozzle part 16. The proximal end portion of the spray channel 32 is constituted by an inside channel 34 provided inside the pressing part 14. The inside channel 34 extends from the distal end of the pressing part 14 in the axial direction, bends downward at the axially middle portion of the pressing part 14 and extends to the outside, and the proximal end of the inside channel 34 opens to the outside as a connection port 36. A pipe-shaped member 40 is connected to the connection port 36 via a flexible tube 38, and in the present practical embodiment, a filter 42 is provided inside the pipe-shaped member 40. The filter 42 is configured such that pressurized gas can pass therethrough, while unwanted bacteria in the pressurized gas cannot pass therethrough. By the pipe-shaped member 40 communicating with the supply source of the pressurized gas, aseptic pressurized gas is supplied to the inside channel 34. On the other hand, the distal side portion of the spray channel 32 is constituted by the spray nozzle part 16 including the soft and hard tubes 26, 28.

Besides, in the middle portion of the spray channel 32, an operating means 44 for switching the spray channel 32 between a communication state and a closed state, and a powder supply mechanism 46 for supplying medical powder into the spray channel 32 are provided. By operating the operating means 44, it is possible to switch between spray and stop of the medical powder.

The operating means 44 includes the pressing part 14 and an open/close valve 48, and transmits the external operating force applied to the pressing part 14 to the open/close valve 48 so as to switch between opening and closing of the open/close valve 48 and hence the spray channel 32. While various known structures can be adopted as the open/close valve 48, the present practical embodiment adopts the structure in which a sealing valve is arranged at the distal end of the inside channel 34, and the fixed nozzle 50 is provided at the distal end side of the sealing valve. By the fixed nozzle 50 being inserted into the sealing valve, the inside channel 34 communicates with the fixed nozzle 50, while by the fixed nozzle 50 being pulled out, the inside channel 34 is placed in a sealed state and closed.

A tubular member 52 extending in the axial direction is provided at the distal end of the pressing part 14, and the sealing valve is held clasped between the pressing part 14 and the tubular member 52 by bonding or the like. Meanwhile, the fixed nozzle 50 provided on the distal end side of the sealing valve is fixed inside the proximal end portion of the body part 20, and extends toward the proximal end side, so that the extending tip end of the fixed nozzle 50 enters the inside of the tubular member 52. Moreover, a spring (not shown) is provided in a compressed state at the proximal end portion of the body part 20 and urges the pressing part 14 toward the proximal end side all the time.

With this configuration, in the initial state, the pressing part 14 is elastically positioned at the moving end on the proximal end side, and the fixed nozzle 50 and the sealing valve are remote from each other at a predetermined distance, so that the open/close valve 48 is held in the closed state. On the other hand, as shown in FIGS. 1 to 4, by pushing the pressing part 14 toward the distal end side against the urging force, the fixed nozzle 50 is inserted into the sealing valve so that the spray channel 32 is brought into the communication state. Besides, by releasing the pushing force applied to the pressing part 14, the pressing part 14 automatically returns to the initial position.

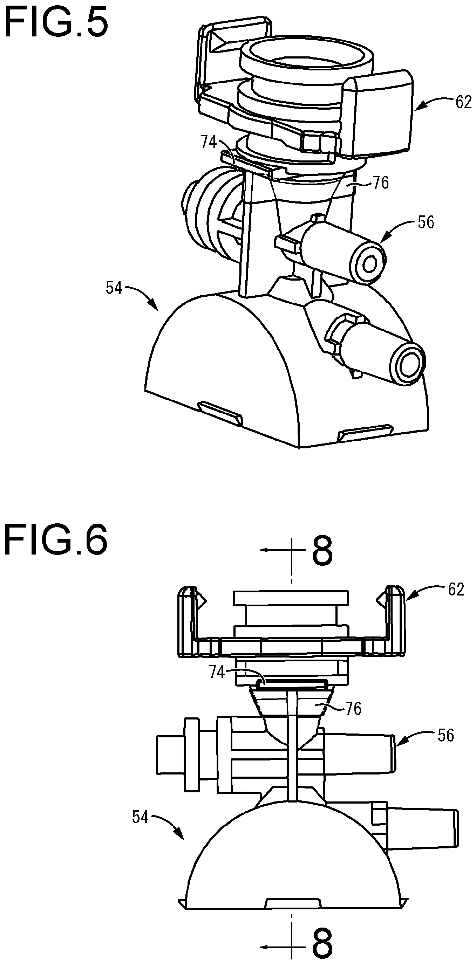

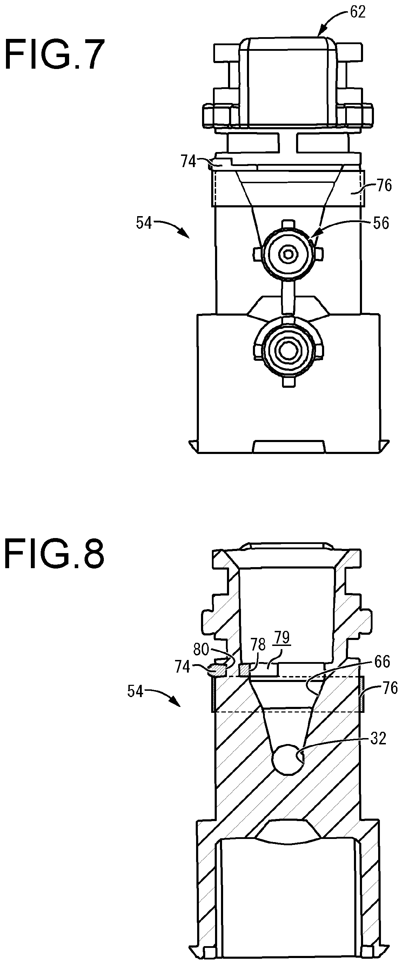

Further, in the body part 20, a retaining member 54 shown in FIGS. 5 to 8 is provided on the distal end side of the fixed nozzle 50. In present practical embodiment, the retaining member 54 is formed of a rigid synthetic resin, and at least a portion constituting a powder channel 66 described later is formed of polycarbonate which is a thermoplastic resin. In the present practical embodiment in particular, in the spray device 10, each member except the soft tube, the O-ring, the spring, etc. is formed of polycarbonate, and the retaining member 54 is also entirely formed of polycarbonate. However, the synthetic resin forming each member of the spray device 10 is not limited to polycarbonate, and, for example, "TRITAN (registered trademark)" manufactured by Eastman Chemical Company, acrylic resin, or the like can be preferably adopted.

A relay channel 56 extending in the axial direction is formed in the retaining member 54, and the distal end of the relay channel 56 is directly or indirectly connected to the proximal end of the soft tube 26, while the proximal end of the relay channel 56 is directly or indirectly connected to the distal end of the fixed nozzle 50. This configuration constitutes the spray channel 32, which extends from the inside channel 34 of the pressing part 14 to the distal end of the spray nozzle part 16 via the fixed nozzle 50 and the relay channel 56. In the present practical embodiment, the proximal end of the relay channel 56 and the distal end of the fixed nozzle 50 are indirectly connected by a plurality of flexible tube pipes, tubular members, or the like.

Meanwhile, a roughly tubular fixing member 58 is fastened externally onto the proximal end portion of the soft tube 26, and the proximal end side of the fixing member 58 is fastened internally to a roughly tubular support member 60. Here, the fixing member 58 and the support member 60 are set so that they can be attached to and detached from each other. Thus, even when the medical powder is gelled by water in the body and clogs the inside of the spray nozzle part 16 so as to disable spraying of the medical powder, the situation can be easily solved by replacing the fixing member 58 and the spray nozzle part 16. Besides, the distal end side of the relay channel 56 is fastened internally to the proximal end side of the support member 60. By so doing, the distal end of the relay channel 56 and the proximal end of the soft tube 26 are indirectly connected. That is, the gas flow path to which the pressurized gas is supplied from the external pressure source is constituted by including the inside channel 34, the fixed nozzle 50, the relay channel 56, and the soft and hard tubes 26, 28, and the pressurized gas flows across the entire length of the spray channel 32.

On the other hand, a container holder 62 exposed upward from the body part 20 is provided to the retaining member 54 serving as the powder supply mechanism 46, and a powder container 64 containing the medical powder is detachably attached to the container holder 62. It should be noted that the powder container 64 of various shapes and sizes are adopted depending on the type or the like of medical powder to be used, and the shape and size of the container holder 62 is also set appropriately depending on the powder container 64 used. In particular, in the present invention, the medical powder to be used is not limited, but for example, in addition to an adhesion preventive material, the present invention can be applied to medical powders such as hemostatic materials and various medicines, and the powder container 64 and the container holder 62 adopted are also appropriately set accordingly. The material of the powder container 64 is not limited in any way, but any conventionally known container material such as glass and rigid synthetic resin can be adopted. In the present practical embodiment, an adhesion preventive material containing PGA (polyglutamic acid) is adopted as the medical powder. PGA is composed of a carboxylic acid, and since electrons are easily transferred, static electricity is likely to be generated. Thus, the effects of the present invention can be more excellently obtained.

Moreover, the retaining member 54 is provided with a powder channel 66 extending from the formation portion of the container holder 62 toward the relay channel 56. In the present practical embodiment, the powder channel 66 extends roughly straightly in the vertical direction. The upper portion of the powder channel 66 on the powder container 64 side extends roughly straightly with a larger inner diameter dimension than that of the lower portion thereof, while the lower portion of the powder channel 66 on the relay channel 56 side decreases in inner diameter dimension toward the relay channel 56. Then, by attaching the powder container 64 to the container holder 62 with its opening part facing downward, the inside of the powder container 64 communicates with the relay channel 56 through the powder channel 66, and the medical powder contained in the powder container 64 is supplied to the relay channel 56. The shape of the powder channel 66 is not limited in any way. The powder channel 66 may extend straightly across the entire length in the lengthwise direction, or may be bent or curved partially or entirely. Further, the powder channel 66 may be formed so as to be inclined with respect to the vertical direction.

Furthermore, the retaining member 54 is equipped with a vibrator mechanism 68. A conventionally known vibrator mechanism such as the one disclosed in, for example, Japanese Unexamined Patent Publication No. JP-A-2012-143502 can be adopted as the vibrator mechanism 68. That is, an eccentric rotor 70 is attached to the lower portion of the retaining member 54 so as to be rotatable about the center axis. Besides, the eccentric rotor 70 is configured to be rotated by fluid pressure applied through a branch channel 72 branched from the distal end side of the fixed nozzle 50. Here, the center of gravity of the eccentric rotor 70 is set eccentric from the rotation center axis. By fluid pressure being applied to a pressure receiving surface of sawtooth form provided on the outer circumferential surface to rotate the eccentric rotor 70 about the rotation center axis, the eccentric rotor 70 vibrates the retaining member 54 and the powder container 64 attached to the retaining member 54 at a frequency corresponding to the rotation cycle. Then, by the powder container 64 being vibrated, the clogging of inside of the powder container 64, the powder channel 66, or the like with the medical powder is prevented, thereby more stably supplying the medical powder to the relay channel 56 as described above.

Here, as shown in FIGS. 5 to 8, the retaining member 54 of the present practical embodiment includes a conductive member 74 having conductivity, and a static eliminator 76 that eliminates electricity by, for example, discharging electricity into the atmosphere (space).

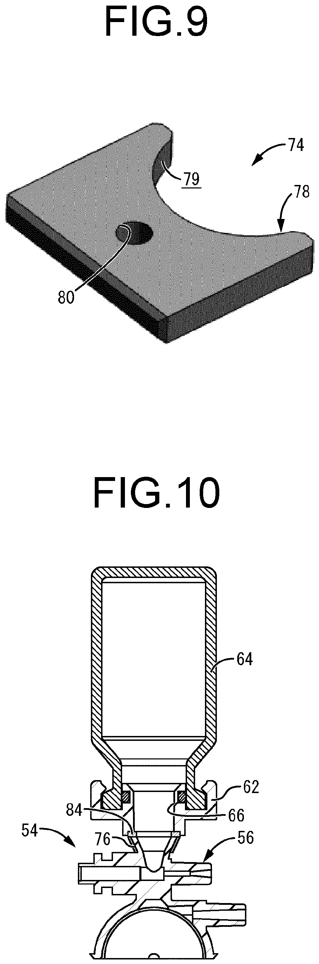

The material of the conductive member 74 is not limited in any way as long as it has conductivity. In the present practical embodiment, the conductive member 74 is made of stainless steel (SUS). However, in addition to metal, conductive resin, conductive rubber or the like can also be adopted. Besides, the shape of the conductive member 74 is not limited in any way either. While a mesh shape or the like may be adopted, in the present practical embodiment, as shown in FIG. 9, the conductive member 74 has a thin rectangular plate shape. In the present practical embodiment, the conductive member 74 is arranged in a state of being roughly embedded in the retaining member 54 in the vertically middle portion of the powder channel 66. Specifically, the conductive member 74 is provided at the boundary part between the upper portion of the powder channel 66, which is large in diameter, and the lower portion thereof, which becomes smaller in diameter toward the lower side. Further, the conductive member 74 is provided with a roughly semicircular notch 78 having a curvature approximately equal to that of the radially inner surface of the powder channel 66, the notch 78 being formed on one side portion of the conductive member 74. A part of the conductive member 74 (particularly a radially inner surface 79 of the notch 78) is exposed to the powder channel 66, and another part of the conductive member 74 (particularly the side portion opposite to the side where the notch 78 is provided) is exposed to the outer peripheral surface of the retaining member 54. In the present practical embodiment, the conductive member 74 includes a through hole 80 for filling the molding material, and the retaining member 54 is formed by insert molding performed with the conductive member 74 set inside the mold. However, for example, the retaining member and the conductive member may be separately formed and assembled later.

Meanwhile, the material of the static eliminator 76 is not limited in any way as long as it can eliminate electricity by, for example, discharging it into the atmosphere, and is made of, for example, a conductive polymer. The method of eliminating electricity by the static eliminator 76 is not limited to discharge into the atmosphere, but in the present practical embodiment, electricity is eliminated by corona discharge. Further, the shape of the static eliminator 76 is not limited in any way either, but in the present practical embodiment, the static eliminator 76 has a roughly tapered tubular shape whose diameter gradually decreases downward. That is, in the present practical embodiment, the sheet-shaped static eliminator 76 is wound around and fixed to the outer peripheral surface of the retaining member 54 at a position corresponding to the lower portion of the powder channel 66. The static eliminator 76 is exposed to an external space 82 outside the powder channel 66 (in the present practical embodiment, the inside space of the body part 20). The static eliminator 76 only needs to be exposed to the space located away from the powder channel 66, and need not be exposed to the space outside the spray device 10. In the present practical embodiment, "DENKITOL (registered trademark in Japan)" manufactured by Japan Vilene Company, Ltd. is used as the static eliminator 76. However, for example, "Electro mesh (TM)" manufactured by Daihyaku Co., Ltd., "Houden-kun paper (TM)" manufactured by JAPACK CO., LTD. or the like can also be adopted.

In the present practical embodiment, the static eliminator 76 is fixed to the outer peripheral surface of the retaining member 54 and is electrically connected to the conductive member 74 by being directly in contact with the lower surface of the portion of the conductive member 74 exposed to the outer peripheral surface of the retaining member 54. The conductive member and the static eliminator may be disposed so as to be remote from each other, and may be electrically connected by being indirectly connected by a member having conductivity.

With respect to the spray device 10 of the present practical embodiment having such a shape, the user hooks his/her fingers on the finger hook parts 22a, 22b and overlap his/her palm, more specifically, the base part of his/her palm side thumb and the operating part 24 of the pressing part 14, so as to grasp the grasping part 12. Then, by pushing the pressing part 14 toward the distal end side with the palm so as to switch the spray channel 32 to the communication state, the user is able to spray the medical powder contained in the powder container 64 from the distal end opening of the spray nozzle part 16. That is, as shown in FIGS. 1 to 4, when the pressing part 14 is pushed toward the distal end side, the open/close valve 48 which is held in the closed state is opened, and the entire length of the spray channel 32 is brought into the communication state, thereby supplying the pressurized gas through the gas flow path. Meanwhile, in association therewith, the pressurized gas is supplied to the vibrator mechanism 68 through the branch channel 72 that branches from the fixed nozzle 50. Accordingly, the eccentric rotor 70 rotates and the vibrating force is continuously generated, and the medical powder within the powder container 64 is delivered into the spray channel 32 little by little, so as to be sprayed to the outside from the distal end opening of the spray nozzle part 16 together with the pressurized gas flowing through the spray channel 32.

On the other hand, when the pushing force of the pressing part 14 is released, the pressing part 14 returns to the initial position by the urging force of the spring provided in the body part 20. Accordingly, the open/close valve 48 held in the open state is closed and the spray channel 32 is brought into the closed state. By so doing, the supply of the pressurized gas into the spray channel 32 is stopped, so that the spray of the medical powder to the outside is also stopped.

That is, the spray channel 32 is switched between the communication state and the closed state by the operating means 44. Specifically, the supply and stop of the pressurized gas in the spray device 10 are switched by the operating means 44. Here, when the spray channel 32 is in the communication state, the medical powder is sprayed, while when the spray channel 32 is in the closed state, the spray of the medical powder is stopped. Thus, the operating means 44 also switches between performing and stopping of spraying the medical powder.

With the medical powder internal spray device 10 of the present practical embodiment having the above-described structure, the conductive member 74 is disposed in an exposed state to the powder channel 66, while the static eliminator 76 is disposed in an exposed state to the external space 82 outside the powder channel 66. Besides, the conductive member 74 and the static eliminator 76 are electrically connected to each other. Thus, static electricity generated by rubbing of the medical powder against the powder container 64 or the powder channel 66, and/or by rubbing among the medical powder itself, will be discharged to the external space 82 outside the powder channel 66 by the static eliminator 76 through the conductive member 74. This prevents the medical powder from sticking to the powder container 64 or the powder channel 66, or from agglomerating due to the action of static electricity, thereby stably spraying the medical powder through the spray channel 32.

In the present practical embodiment in particular, the retaining member 54 is equipped with the vibrator mechanism 68, and the vibration applied by the vibrator mechanism 68 stirs the medical powder in the powder container 64 and in the powder channel 66. By so doing, the medical powder further rubs against the powder container 64 and the powder channel 66, and/or rubs among the medical powder itself, so that more static electricity is generated. Therefore, by eliminating the static electricity in the way as described above, the medical powder can be sprayed more stably.

Moreover, in the present practical embodiment, since the retaining member 54 constituting the powder channel 66 is formed of polycarbonate which is a thermoplastic resin, the surface of the powder channel 66 is likely to be charged. As a result, static electricity is even more likely to be generated by the powder channel 66 and the medical powder rubbing against each other. However, by eliminating the static electricity in the way as described above, the medical powder can be sprayed more stably.

Furthermore, in the present practical embodiment, an adhesion preventive material containing PGA is adopted as the medical powder. In the present practical embodiment in particular, from the viewpoint of stability, it is preferable that the medical powder is contained in the powder container 64 in a dry state and sprayed from the spray channel 32. However, since PGA contains a carboxylic acid, electrons are easily transferred, and static electricity is even more likely to be generated in a dry state. Even in such a case, by eliminating the static electricity in the way as described above, the medical powder can be sprayed more stably.

Next, FIG. 10 shows a retaining member 54, which constitutes an internal spray device for medical powder according to a second practical embodiment of the present invention, with a powder container 64 attached. In the present practical embodiment, the shape of the retaining member 54 is roughly the same as that in the first practical embodiment, but the shape of a conductive member 84 provided to the retaining member 54 is different from that in the first practical embodiment. In the present practical embodiment, elements like those in the first practical embodiment shall be designated by like reference numerals and will not be discussed in detail.

That is, the conductive member 84 of the present practical embodiment has a roughly ring shape, and the conductive member 84 is arranged in a state of being roughly embedded in the retaining member 54 in the vertically middle portion of a powder channel 66. With this configuration, a part of the conductive member 84 (particularly the radially inner surface thereof) is exposed to the powder channel 66, and another part of the conductive member 84 (particularly the outer peripheral portion of the lower surface thereof) is exposed to the outer peripheral surface of the retaining member 54.

Besides, similarly to the preceding first practical embodiment, a sheet-shaped static eliminator 76 is wound around and fixed to the outer peripheral surface of the retaining member 54 at a position corresponding to the lower portion of the powder channel 66. With this configuration, on the outer peripheral surface of the retaining member 54, the exposed portion of the conductive member 84 and the static eliminator 76 are in direct contact and are electrically connected with each other.

Therefore, even when the conductive member 84 is shaped as described above, the static electricity generated in the powder channel 66 is discharged to an external space 82 outside the powder channel 66 through the conductive member 84 and the static eliminator 76. Thus, the same effect as that of the preceding first practical embodiment can be obtained.

Although the practical embodiments of the present invention have been described above, the present invention is not limitedly interpreted based on the specific description in the practical embodiments.

For example, in the preceding practical embodiment, the conductive members 74, 84 have a roughly plate shape or a roughly ring shape and are arranged in a state of being roughly embedded in the retaining member 54, and the static eliminator 76 has a roughly tapered tubular shape and is fixed to the outer peripheral surface of the retaining member 54. However, the positions where the conductive member and the static eliminator are disposed are not limited in any way as long as the conductive member is exposed to the powder channel and the static eliminator are exposed to the space outside the powder channel so as to discharge. That is, for example, the conductive member may be arranged by fixing at a portion in contact with the powder, such as the radially inner surface of the retaining member constituting the powder channel, or may be provided to the powder channel within the powder container in addition to or in place of the powder channel of the preceding practical embodiment. Further, the static eliminator may be provided on the radially inner surface or the radially outer surface of the body part in place of or in addition to the outer peripheral surface of the retaining member, and may be electrically connected to the conductive member through an appropriate member having conductivity. That is, the conductive member and the static eliminator may be fixed to each other as needed, for example, in a state of direct contact, or may be fixed to each other by a member having conductivity in a contact state or in a non-contact state. In addition, for example, by forming an arbitrary powder channel forming member, which is attached so as to be exposed to the external space, with a conductive member such as metal, it is possible to implement the conductive member and the static eliminator that are electrically connected. That is, the conductive member and the static eliminator may be constituted by a single member.

Moreover, in the preceding practical embodiment, the spray device 10 has a roughly syringe shape overall, but the shape of the spray device is not limited in any way. That is, the spray device may adopt the shape described in any of, for example, JP-A-2017-51545 (above-mentioned Patent Document 1), Japanese Unexamined Patent Publication No. JP-A-2016-22259, Japanese Unexamined Patent Publication No. JP-A-2014-140577, and the like, in addition to the shape described in the preceding practical embodiment.

Furthermore, in the preceding practical embodiment, the spray nozzle part 16 is constituted by including the soft tube 26 so that the spray direction of the medical powder can be easily changed. However, for example, the spray nozzle part 16 may be constituted only by a hard tube, and the mechanism for changing the spray direction is not essential. Also, in the preceding practical embodiment, the fixing member 58 and the support member 60 are detachable from each other, and can be replaced when, for example, the spray nozzle part 16 is clogged. However, the spray nozzle part may be integrally formed with the body part so that it cannot be replaced. In that case, it is preferable to separately provide a mechanism for removing the clogging caused by the medical powder.

Additionally, in the present invention, the vibrator mechanism for vibrating the powder channel is not essential. That is, the medical powder may be supplied from the powder container to the spray channel through the powder channel by, for example, the action of gravity.

Besides, in the preceding practical embodiment, the powder container 64, which is a separate member from the spray device 10, is additionally attached, but it would also be acceptable to form a powder container capable of containing medical powder integrally with the spray device. Alternatively, the medical powder may be supplied from an external powder container. Even in such a case, the channel extending from the portion containing the medical powder to the spray channel serves as the powder channel.

In addition, whereas in the present invention, the pressurized gas is supplied from the outside, a cylinder of the pressurized gas or the like may be incorporated inside the spray device, for example.

### KEYS TO SYMBOLS

10: internal spray device for medical powder, 32: spray channel, 66: powder channel, 68: vibrator mechanism, 74, 84: conductive member, 76: static eliminator, 82: external space

## Claims

1. An internal spray device for medical powder including a spray channel where pressurized gas flows and a powder channel through which the medical powder is supplied to the spray channel so that the medical powder is sprayed together with the pressurized gas via the spray channel into a body, the internal spray device being **characterized in that**:
the internal spray device further includes a conductive member exposed to the powder channel and a static eliminator exposed to an external space outside the powder channel, and
the conductive member and the static eliminator are electrically connected to each other.

2. The internal spray device according to claim 1, wherein the internal spray device further includes a vibrator mechanism vibrating the powder channel.

3. The internal spray device according to claim 1 or 2, wherein a member constituting the powder channel is formed of a thermoplastic resin.

4. The internal spray device according to any one of claims 1-3, wherein the medical powder comprises an adhesion preventive material.
